# EUROPEAN PATENT SPECIFICATION

(11) **EP 3 773 858 B1**
(45) Date of publication and mention of the grant of the patent: **29.11.2023**
(21) Application number: 19786231.1
(22) Date of filing: 11.04.2019
(51) Int. Cl.: A61M 25/10, A61M 29/02, A61L 29/04

(54) **MULTI-STAGE BALLOON CATHETER SYSTEMS**
MEHRSTUFIGE BALLONKATHETERSYSTEME
SYSTÈMES DE CATHÉTER À BALLONNET À PLUSIEURS ÉTAGES

(30) Priority: 13.04.2018 US 201862657203 P
(43) Date of publication of application: 17.02.2021
(73) Proprietor: MERIT MEDICAL SYSTEMS, INC., South Jordan, UT 84095 (US)
(72) Inventor: HOPKINSON, Aaron, Herriman, Utah 84096 (US); WIERSDORF, Jason, West Jordan, Utah 84081 (US)
(74) Representative: CSY London
(86) International application number: PCT/US2019/026930
(87) International publication number: WO 2019/200062

(56) References cited:
- WO-A2-2016/149653
- US-A- 4 758 223
- US-A- 5 545 133
- US-A- 5 545 133
- US-A1- 2006 224 113
- US-A1- 2009 076 439
- US-A1- 2013 165 903
- US-A1- 2013 165 903

## Description

### RELATED APPLICATIONS

This application claims priority to United States Provisional Application No. 62/657,203, filed on April 13, 2018, and titled "MULTI-STAGE BALLOON CATHETER SYSTEMS AND METHODS".

### TECHNICAL FIELD

The present disclosure relates generally to catheters and related medical devices. More specifically, the present description relates to balloon catheter assemblies and methods of use.

### BRIEF DESCRIPTION OF THE PRIOR ART

US2013/165903 discloses a catheter body comprising an inflation lumen, a fluid connector, and a balloon.

### SUMMARY OF THE INVENTION

The invention is set out in the appended set of claims.

### BRIEF DESCRIPTION OF THE DRAWINGS

The embodiments disclosed herein will become more fully apparent from the following description and appended claims, taken in conjunction with the accompanying drawings. While various aspects of the embodiments are presented in drawings, the drawings depict only typical embodiments, which will be described with additional specificity and detail through use of the accompanying drawings in which:
FIG. 1 is a top view of a balloon dilation system, comprising a balloon catheter assembly.
FIG. 2 is a perspective view of the balloon catheter assembly of FIG. 1.
FIG. 3 is a cross-sectional view of the balloon catheter assembly of FIG. 2.
FIG. 4A is a plan view of the balloon dilation system of FIG. 1 inserted into a strictured body lumen.
FIG. 4B is a plan view of the balloon dilation system of FIG. 1 inserted into the strictured body lumen where a balloon is expanded to a Stage 1 configuration.
FIG. 4C is a plan view of the balloon dilation system of FIG. 1 inserted into the strictured body lumen where the balloon is expanded to a Stage 2 configuration.
FIG. 4D is a plan view of the balloon dilation system of FIG. 1 inserted into the strictured body lumen where the balloon is expanded to a Stage 3 configuration.
FIG. 4E is a plan view of the balloon dilation system of FIG. 1 inserted into the strictured body lumen where the balloon is expanded to a Stage 4 configuration.
FIG. 5 is a table depicting balloon catheter configurations and corresponding expanded balloon diameters and pressures.

### DETAILED DESCRIPTION

In some medical procedures, a balloon or other medical device passes through and emerges distal of an elongate channel, such as a working channel or lumen of an elongate device, such as an endoscope or a catheter. There, disposed distal of the working channel, the balloon or other medical device may be expanded (e.g., inflated in the case of a balloon) for some reason, such as to widen a passageway, secure a stent, collect emboli, etc. In some procedures the balloon is inflated or expanded in stages to widen the passageway incrementally, for example to minimize trauma to surrounding tissue. In some instances, each stage of inflation correlates to a discrete balloon diameter. In some circumstances, the balloon may be incrementally expanded to three stages. In other circumstances, the balloon may be incrementally expanded to four stages. In yet other circumstances, the balloon may be incrementally expanded to five or more stages.

Some of the medical devices and/or medical systems disclosed herein comprise a balloon dilation catheter configured to be incrementally expanded to four or more stages. The medical system may also comprise an elongate device, such as an endoscope and/or a balloon inflation device. The medical devices and/or medical systems may be used to treat a stricture of an esophagus. In other circumstances, the medical devices and/or medical systems may be used to treat strictures of other body lumens, such as blood vessels, urinary tract, airway, etc.

The various embodiments disclosed herein generally relate to balloon catheters. More specifically, the various embodiments relate to multi-stage balloon catheter systems, including those comprising fixed wire multi-stage balloon catheter assemblies, an endoscope, and a balloon inflation device, and related methods.

It will be appreciated that various features are sometimes grouped together in a single embodiment, figure, or description thereof for the purpose of streamlining the disclosure. Many of these features may be used alone and/or in combination with one another. It will further be appreciated that many of the features disclosed herein may be used in conjunction with other catheter assemblies presently known or hereafter developed.

Embodiments may be understood by reference to the drawings, wherein like parts are designated by like numerals throughout. It will be readily understood that the components of the present disclosure, as generally described and illustrated in the drawings herein, could be arranged and designed in a wide variety of different configurations. Thus, the following more detailed description of the embodiments of the apparatus is not intended to limit the scope of the disclosure, but is merely representative of possible embodiments of the disclosure. In some cases, well-known structures, materials, or operations are not shown or described in detail. While the various aspects of the embodiments are presented in drawings, the drawings are not necessarily drawn to scale unless specifically indicated.

The phrases "connected to," "coupled to," and "in communication with" refer to any form of interaction between two or more entities, including but not limited to mechanical, electrical, magnetic, electromagnetic, fluid, and thermal interaction. Two components may be coupled to each other even though they are not in direct contact with each other. For example, two components may be coupled to each other through an intermediate component.

The terms "proximal" and "distal" refer to opposite ends of a medical device, including the devices disclosed herein. As used herein, the proximal portion of a medical device is the portion nearest a practitioner during use, while the distal portion is a portion at the opposite end. For example, the proximal end of a balloon catheter assembly is defined as the end closest to the practitioner during insertion or utilization of the balloon catheter assembly. The distal end is the end opposite the proximal end, along the longitudinal direction of the balloon catheter assembly.

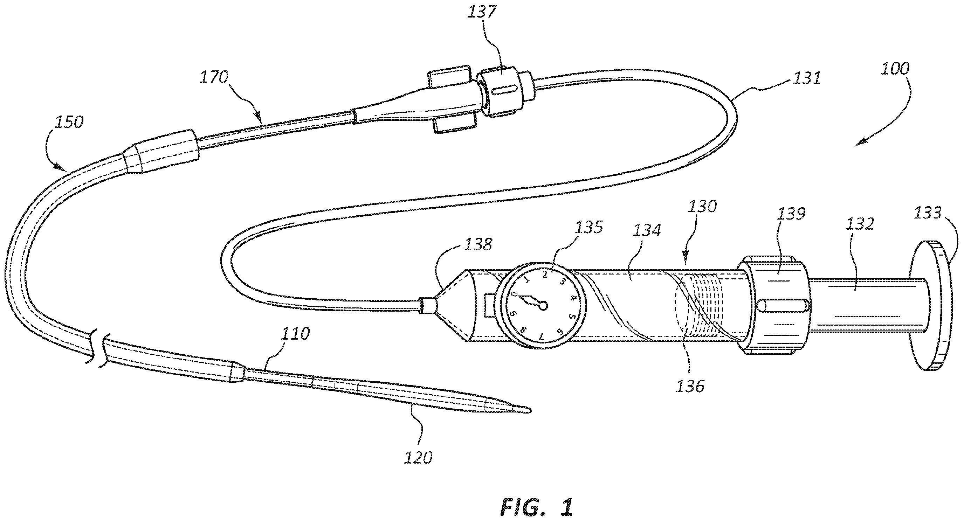

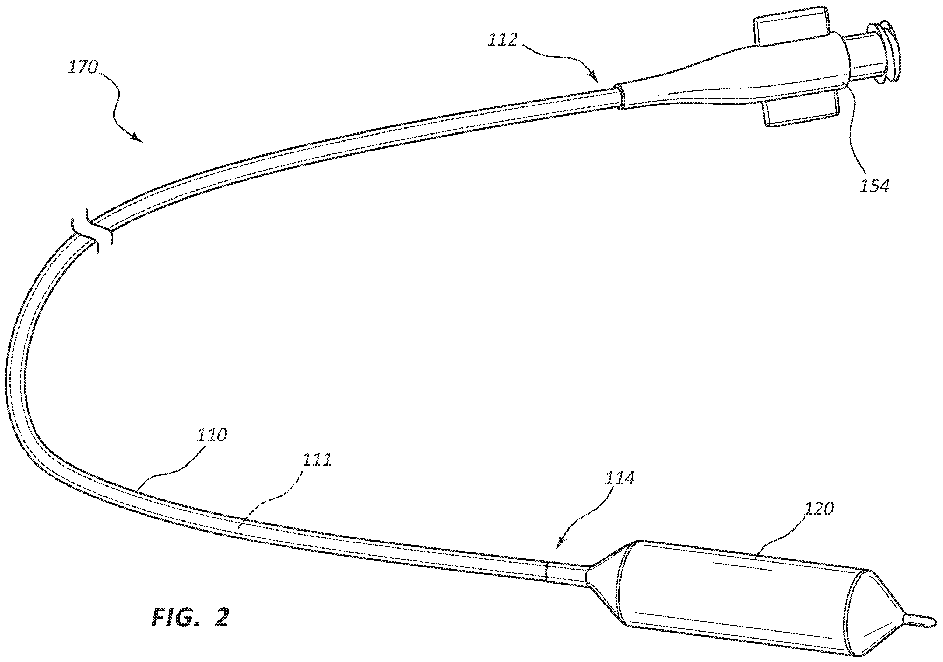

FIG. 1 illustrates a balloon dilation system 100 comprising a balloon catheter assembly 170, a balloon inflation device 130, and an elongated device 150. FIG. 2 illustrates an embodiment of the balloon catheter assembly 170. The balloon catheter assembly 170 may comprise a hub 154, a catheter body 110, and an inflation balloon 120. The catheter body 110 may be configured to provide a passageway for components and/or substances between at least the hub 154 and the inflation balloon 120. For example, in the embodiment of FIG. 1, a distal end 114 of the catheter body 110 is coupled to the inflation balloon 120 and a proximal end 112 of the catheter body 110 is coupled to the hub 154.

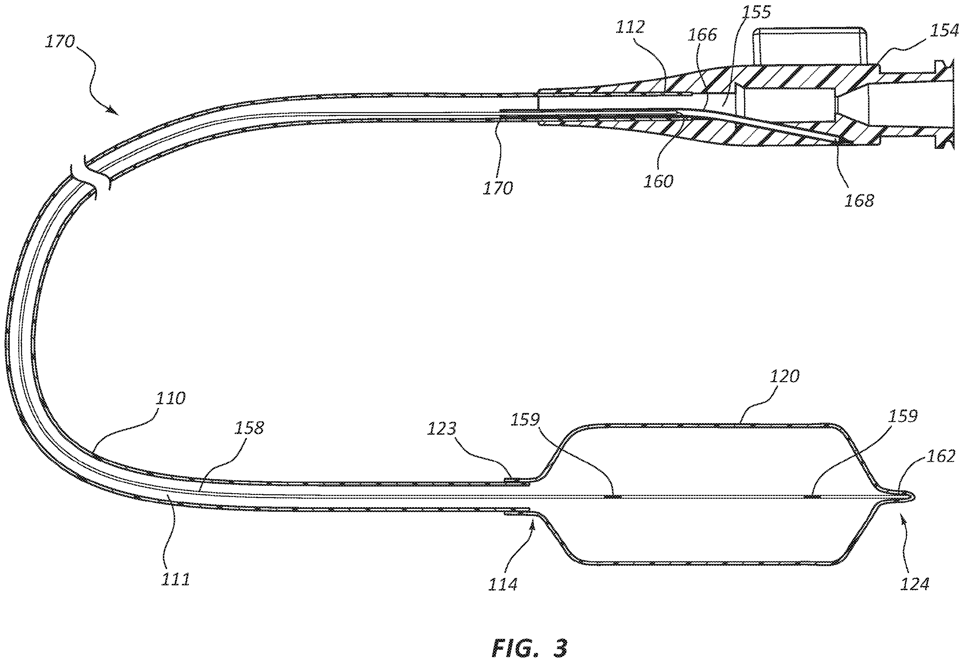

In the illustrated embodiment with reference to FIGS. 2 and 3, the catheter body 110 comprises a lumen 111 extending longitudinally therethrough. For example, the lumen 111 can be configured to serve as a passageway through which an inflation fluid (e.g., a gas or a liquid) may be introduced into and/or withdrawn from the inflation balloon 120. In such embodiments, the lumen 111 may be referred to as an inflation/deflation lumen. In some other embodiments, the catheter body 110 may comprise a plurality of lumens extending longitudinally therethrough. The catheter body 110 may be configured in a range of diameters to accommodate a range of diameters of the body lumen into which the balloon catheter assembly 170 may be inserted, such as the esophagus. The diameter of the catheter body 110 may range from five French to ten French, from six French to nine French, and from seven French to eight French.

In certain embodiments, the catheter body 110 comprises a polymeric material, such as polyurethane, polyethylene, polyethylene terephthalate, nylon, polyether block amide, etc. The polymeric material may be extruded to form the catheter body 110 using one or more extrusion techniques. The catheter body 110 may also be referred to as catheter tubing, an elongated tubular member, a tubular member, or a first tubular member.

The inflation balloon 120 is disposed at a distal portion of the balloon catheter assembly 170. The inflation balloon 120 may be formed from any suitable polymeric material, such as nylon, polyether block amide, polyurethane, etc. using any suitable technique, such as blow molding, extrusion, dip molding, injection molding, etc. The inflation balloon 120 may be transparent or translucent such that tissue surrounding the inflation balloon 120 may be easily visualized during a dilation procedure. The balloon 120 may be generally cylindrical in shape with tapered proximal distal ends. The balloon 120 may be pre-formed to a plurality of diameters such that a plurality of balloon dilation catheter 110 configurations may be provided as will be discussed below. An interior of the inflation balloon 120 may be in fluid communication with the lumen 111 of the catheter body 110 (i.e., the inflation/deflation lumen). For example, inflation fluid may flow between the inflation/deflation lumen 111 of the catheter body 110 and the inflation balloon 120, or the interior thereof, during both inflation and deflation procedures, as discussed further below.

The balloon 120 may be configured to be inflated or expanded to discrete diameters corresponding to discrete internal pressures. The diameter of the balloon 120, when expanded, may range from about two mm to about twenty-five mm, from about three mm to about twenty-three mm, and from about five mm to about twenty-one mm. The balloon 120 may be configured to be expanded with pressures ranging from about one atm to about fifteen atm, from about two atm to about thirteen atm, and from about two atm to about twelve atm. The balloon 120 may be expanded to at least four stages, to at least five stages, and to more than five stages where each stage correlates to a discrete diameter. Further, the balloon 120 can be configured such that defined discrete pressures correlate with defined discrete diameters of the balloon 120 at each stage.

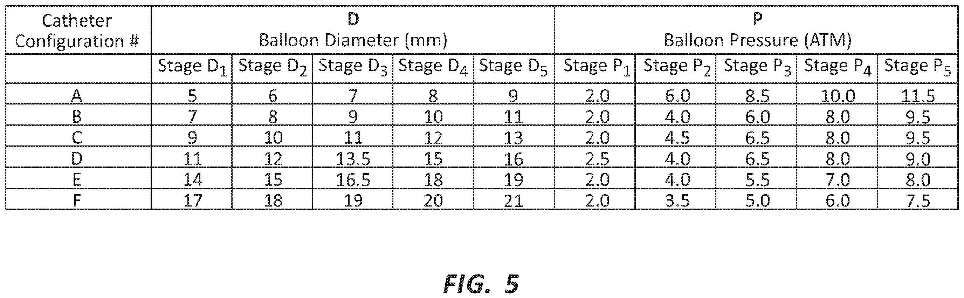

As shown in FIG. 5, the balloon catheter assembly 170 may be provided in a plurality of configurations A-F. Each configuration may provide a balloon 120 configured to be expanded to at least five stages 1-5. Each stage is defined by a predefined discrete balloon diameter D at a correlated predefined discrete pressure P. For example, Catheter Configuration A may be expanded to Stage 1 where the balloon diameter D₁ is five mm at a pressure P₁ within the balloon 120 of 2.0 atm, to Stage 2 where the balloon diameter D₂ is six mm at a pressure P₂ of 6.0 atm, to Stage 3 where the balloon diameter D₃ is seven mm at a pressure P₃ of 8.5 atm, to Stage 4 where the balloon diameter D₄ is eight mm at a pressure P₄ of 10.0 atm, and to Stage 5 where the balloon diameter D₅ is nine mm at a pressure P₅ of 11.5 atm. In use, expansion of the balloon 120 to the various stages is dependent upon the stricture or stenosis being treated. For example, the balloon 120 may be expanded to Stage 1, to Stages 1 and 2, to Stages 1, 2, and 3, to Stages 1, 2, 3, and 4, or to Stages 1, 2, 3, 4, and 5 as determined by the practitioner performing the balloon dilation procedure. The practitioner may stop balloon 120 expansion at any stage dependent upon the condition of the tissue being dilated. For example, the balloon 120 may be expanded only to Stage 3 because the practitioner observed blanching of the dilated tissue which may be an indication of the tissue reaching a failure point.

In some embodiments, the information of the catheter configurations A-F may be provided to an operator of the balloon dilation system 100 on a label (not shown). The label may display information regarding a single catheter configuration, such as configuration A, B, C, D, E, or F. In other embodiments, the label may display information for all of the catheter configurations A-F. The label may be coupled to the balloon catheter assembly 170 such that the correlation of the predefined discrete catheter diameters with the predefined discrete balloon diameters can be conveniently noted by the operator. In some embodiments, the label may be included with packaging materials, such as an Instructions for Use label.

FIG. 3 is a cross-sectional view of the balloon catheter assembly 170 of FIG. 2. As discussed above, the balloon catheter assembly 170 comprises the inflation balloon 120, the catheter body 110, and the hub 154. As further shown, the proximal end 112 of the catheter body 110 is coupled to the hub 154, and the distal end 114 of the catheter body 110 is coupled to the inflation balloon 120. The balloon catheter assembly 170 further comprises a support wire 158 and a sleeve 166.

In some embodiments, the support wire 158 is at least partially disposed within the lumen 111 of the catheter body 110. For example, in the illustrated embodiment, the support wire 158 extends longitudinally within the lumen 111 of the catheter body 110 and into the inflation balloon 120, or the interior of the inflation balloon 120. In such embodiments, the support wire 158 is configured to add increased rigidity and/or stiffness to the catheter body 110 and/or the inflation balloon 120, which may aid in insertion of the balloon catheter assembly 170 into a working lumen (e.g., an endoscope) and/or a body lumen during use by a practitioner. As further illustrated, a distal end 162 of the support wire 158 may be coupled to, fixedly coupled to, or attached to the distal end 124 of the inflation balloon 120. The support wire 158 may comprise radiopaque markers 159 coupled to the support wire 158 between the proximal and distal ends of the balloon 120.

A proximal end 160 of the support wire 158 may be at least partially disposed within the sleeve 166. In some embodiments, the proximal end 160 of the support wire 158 may be operatively coupled to the hub 154 and/or the sleeve 166 such that the support wire 158 transfers distally oriented forces exerted on the hub 154 but not proximally oriented forces exerted on the hub 154 (*i*.*e*., distally and/or proximally oriented forces exerted by a practitioner on the hub 154 during use of the balloon catheter assembly 170, such as during a medical procedure, as described in more detail below). In some other embodiments, the proximal end 160 of the support wire 158 can be fixedly coupled to the hub 154. In various embodiments, the illustrated balloon catheter assembly 170 may be referred to as a fixed wire balloon catheter assembly, as the support wire 158 may not be configured, nor intended, to be removed from the balloon catheter assembly 170.

The support wire 158 may be operatively coupled to the balloon catheter assembly 170 such that the support wire 158 does not exert a longitudinally compressive force on the inflation balloon 120 and such that the support wire 158 longitudinally supports the inflation balloon 120 when the inflation balloon 120 is subjected to longitudinally compressive forces. For example, the support wire 158 of the balloon catheter assembly 170 may be configured such that the inflation balloon 120 resists longitudinal compression upon insertion or passage of the inflation balloon 120 into or through a delivery lumen and/or a body lumen.

Referring again to FIG. 1, the balloon inflation device 130 may comprise a syringe body 134, a plunger 132, a pressure gauge 135, a plunger handle 133, and a fluid delivery tube 131. The syringe body 134 may be formed of a generally cylindrical hollow tube configured to receive the plunger 132. The syringe body 134 may include an inlet/outlet port 138 located adjacent a distal end of the syringe body 134. In some embodiments, a coupling member 139 may be coupled to the syringe body 134 adjacent a proximal end of the syringe body 134. The coupling member 139 may include a center aperture configured to allow the plunger 132 to pass through the coupling member 139 into the syringe body 134. Further, the coupling member 139 may include coupling member threads configured to selectively couple the coupling member 139 to threads of the plunger 132. For example, the coupling member 139 may comprise a polymeric nut at the proximal end of the syringe body 134.

The plunger 132 may be configured to be longitudinally displaceable within the syringe body 134. The plunger 132 may be comprised of a plunger shaft coupled to a plunger tip 136 at the distal end of the plunger shaft. The plunger shaft may also be coupled to the handle 133 at the proximal end of the plunger shaft, with the plunger shaft spanning the distance between the plunger seal 136 and the handle 133.

The handle 133 may be configured to be graspable by a user. In certain embodiments, the handle 133 may be configured such that the user may manipulate the position of the plunger 132 by manipulating the handle 133 through rotation or axial translation of the handle 133.

The fluid delivery tube 131 may be coupled to the inlet/outlet port 138 such that a lumen of the fluid delivery tube 131 is in fluid communication with the syringe body 134. The fluid delivery tube 131 may be configured to withstand high fluid pressures. In some embodiments, the fluid delivery tube 131 may comprise a braided structure within a wall. The fluid delivery tube 131 may further comprise a fluid connector 137 coupled to a distal end. The fluid connector 137 may be configured to couple to the hub 154 of the balloon catheter assembly 170 such that the fluid connector is in fluid communication with the hub 154.

The balloon inflation device may further comprise a pressure gage 135 coupled to the syringe body 134. The pressure gauge 135 may be configured to measure pressure within the syringe body 134. Additionally, because of the substantially incompressibility of fluid, the pressure gauge 135 may indicate the pressure within the balloon 120. Indicia may be disposed on the face of the pressure gauge 135. The indicia may indicate the pressure within the syringe body 134 and the balloon 120 in units of pounds per square inch (psi), kilopascal (kPa), atmospheres (atm), etc. The pressure gauge 135 may be an analog gauge or a digital gauge.

With continued reference to FIG. 1, the balloon dilation system 100 may further comprise an elongate device 150. In some embodiments, the elongate device 150 is an endoscope. The elongate device 150 may comprise a body 151 and a working lumen 152 longitudinally disposed within the body 151. In some embodiments, the body 151 may comprise a plurality of lumens configured for disposing of a vision member, a lighting member, fluid delivery, suction, etc. The working lumen 152 can be sized to allow for passage of the balloon 120 and catheter body 110 of the balloon catheter assembly 170.

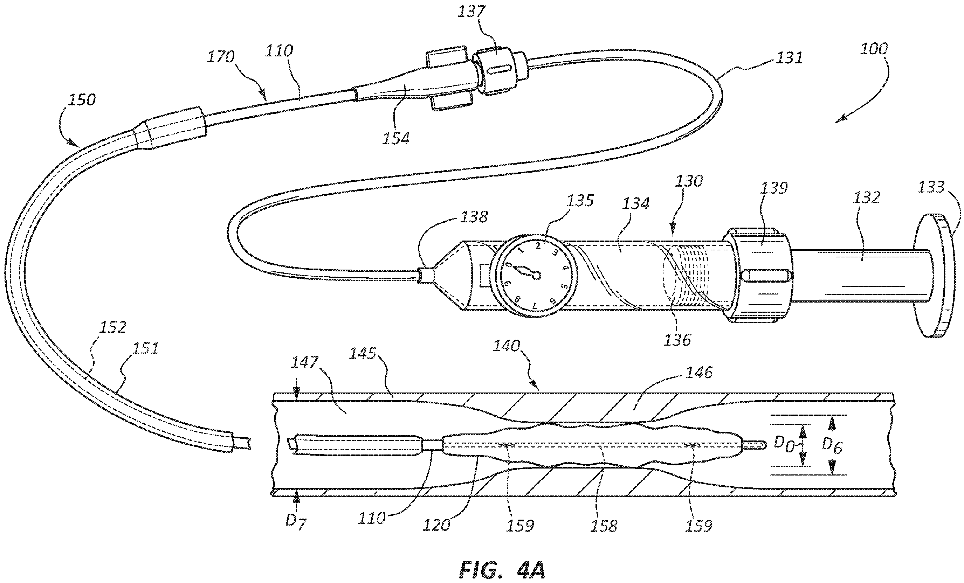

The balloon dilation system 100 may be utilized to treat a stenosis or stricture of a lumen within the body of a patient. For example, the balloon dilation system 100 may be used to treat an esophageal stricture. Referring to FIGS. 4A-4E, a method of treating an esophageal stricture 146 with the balloon dilation system 100 is illustrated. As shown in FIG. 4A, the elongated device 150 (e.g., an endoscope) may be inserted into a lumen 147 defined by a wall 145 of the esophagus 140 of the patient using any suitable technique. A distal end of the elongated device 150 may be positioned proximal to the stricture 146 to be dilated. The balloon catheter assembly 170 that is configured with a non-expanded balloon diameter D₀ approximately equivalent to or less than an internal diameter D₆ of the non-dilated stricture 146 and a Stage 4 balloon diameter D₄ approximately equivalent to a natural diameter D₇ of the esophagus 140 may be selected by the practitioner.

The selected balloon catheter assembly 170 may be inserted into a proximal end of the working lumen 152 and passed through the elongated device 150 such that the distal end 114 of the catheter body 110 and the balloon 120 are disposed distal of the distal end of the elongated device 150. The balloon 120 may be further positioned through the stricture 146 such that the balloon 120 is longitudinally centered within the stricture 146. The balloon 120 may be at diameter D₀ which may be equivalent to or less than the diameter D₆ of the non-dilated stricture 146. The radiopaque markers 159 on the support wire 158 may be visualized using any suitable imaging system, such as X-ray, fluoroscopy, etc., to facilitate proper positioning of the balloon 120. The support wire 158 may prevent axial compression of the balloon 120 during insertion of the balloon catheter assembly 170 through the elongated device 150 and stricture 146. The fluid connector 137 of the inflation device 130 may be coupled to the hub 154 such that a fluid tight seal is created.

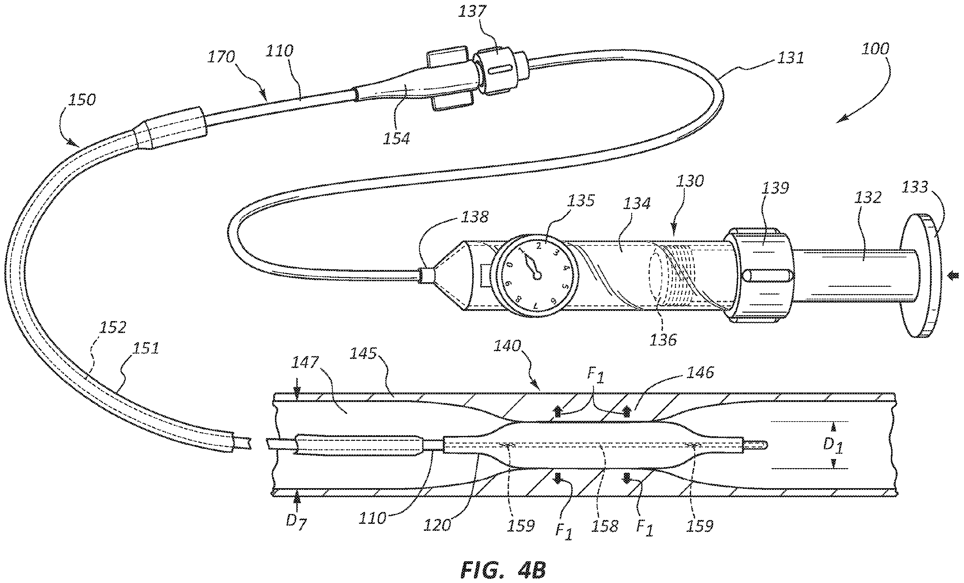

With reference to FIG. 4B, the balloon 120 may be expanded to Stage 1 with a predefined diameter of D₁ by actuating the balloon inflation device 130 to the Stage 1 predefined pressure P_{1,} as indicated and observed on the pressure gauge 135. The balloon inflation device 130 may be actuated either by rotating the handle in a clockwise direction or by axially displacing the handle such that the plunger 132 and plunger tip 136 are displaced distally. As the plunger tip 136 is displaced, fluid within the syringe body 134 flows from the syringe body 134, through the inlet/outlet port 138, through the fluid delivery tube 131, through the connector 137, through the hub 154, through the lumen 111 of the catheter body 110, and into the balloon 120. Displacement of the plunger tip 136 may continue until the pressure gauge 135 reads the Stage 1 pressure P₁. At the Stage 1 predefined pressure P₁, the predefined balloon diameter is Stage 1 D₁ and the balloon 120 may be in contact with the stricture 146 such that an outwardly directed radial force F₁ is applied to the stricture 146. As the diameter of the balloon 120 increases, the outwardly directed radial force applied to the stricture 146 increases, resulting in dilation of the stricture 146 to a diameter substantially equivalent to the expanded balloon diameter.

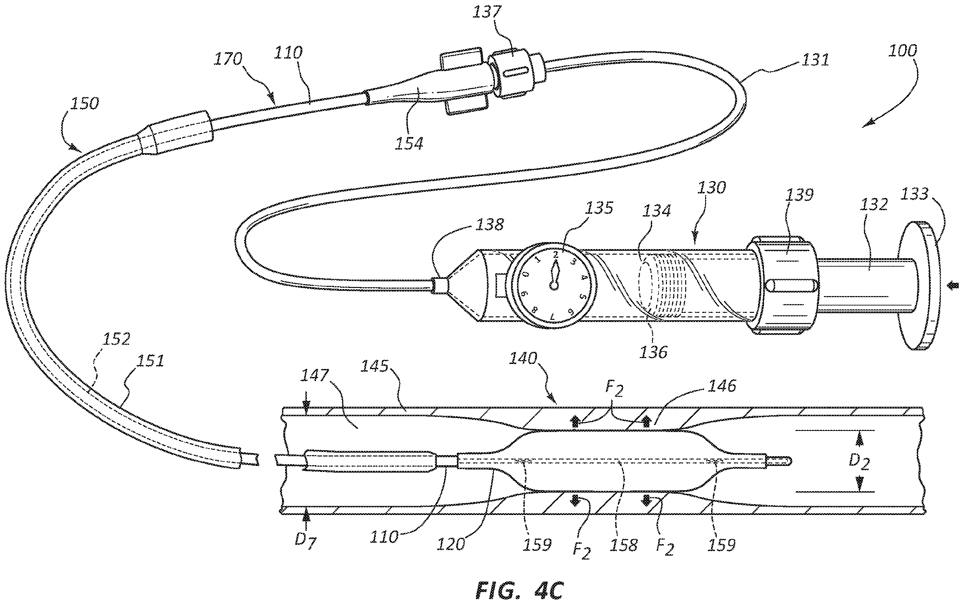

As illustrated in FIG. 4C, the balloon 120 may be expanded to Stage 2 with a predefined diameter of D₂ by continued actuation of the balloon inflation device 130 to the predefined pressure P₂ as indicated and observed on the pressure gauge 135. At the Stage 2 predefined pressure P₂, the balloon diameter is the predefined Stage 2 D₂ and the balloon 120 may be applying outwardly directed radial force F₂ to the stricture 146.

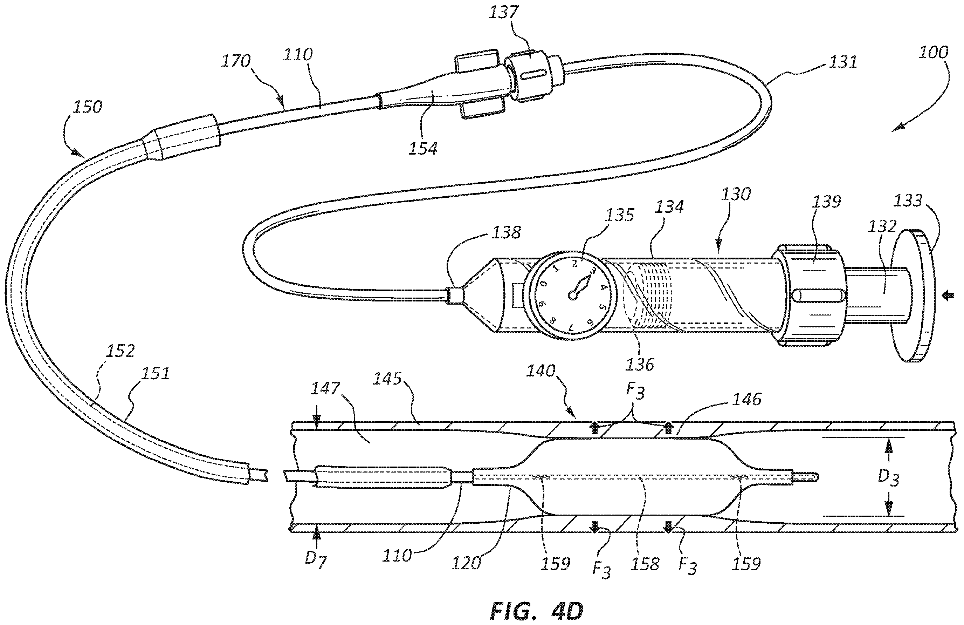

As illustrated in FIG. 4D, the balloon 120 may be expanded to Stage 3 with a predefined diameter of D₃ by continued actuation of the balloon inflation device 130 to the predefined pressure P₃ as indicated and observed on the pressure gauge 135. At the Stage 3 predefined pressure P₃, the balloon diameter is the predefined Stage 3 D₃ and the balloon 120 may be applying outwardly directed radial force F₃ to the stricture 146.

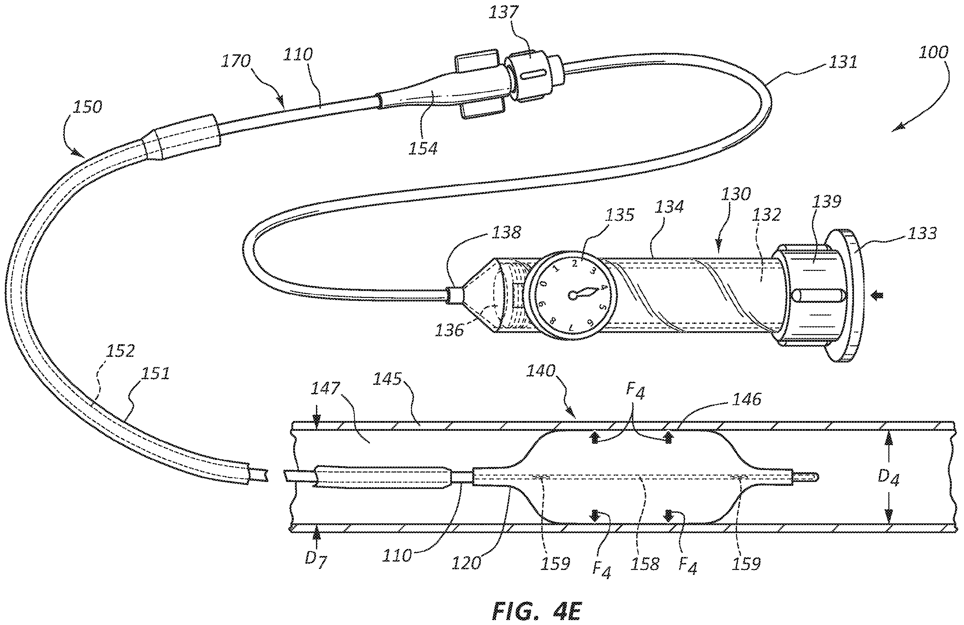

As illustrated in FIG. 4E, the balloon 120 may be expanded to Stage 4 with a predefined diameter of D₄ by continued actuation of the balloon inflation device 130 to the predefined pressure P₄ as indicated and observed on the pressure gauge 135. At the predefined Stage 4 pressure P₄, the balloon diameter is the predefined Stage 4 D₄ and the balloon 120 may be applying outwardly directed radial force F₄ to the stricture 146.

In some embodiments, the balloon 120 may be further expanded to Stage 5 defining a larger diameter than the Stage 4 D₄ balloon diameter. In other embodiments, the balloon 120 may be expanded to Stage 6 or more where each stage defines a larger balloon diameter than a previous stage.

Balloon dilation at each stage may be held at the discrete pressure for approximately thirty seconds and repeated multiple times until the stricture 146 is dilated to the diameter of the expanded balloon at each stage. The balloon inflation device 130 may be used to deflate the balloon 120 such that the balloon catheter assembly 170 may be removed from the esophagus 140 and the elongated member 150. Balloon deflation may be accomplished by rotating the handle 133 in a counterclockwise direction or axially displacing the handle in a proximal direction such that the plunger 132 and plunger tip 136 are displaced proximally and fluid is drawn into the syringe body 134.

References to approximations are made throughout this specification, such as by use of the term "substantially." For each such reference, it is to be understood that, in some embodiments, the value, feature, or characteristic may be specified without approximation. For example, where qualifiers such as "about" and "substantially" are used, these terms include within their scope the qualified words in the absence of their qualifiers. For example, where the term "substantially straight" is recited with respect to a feature, it is understood that in further embodiments, the feature can have a precisely straight configuration.

Reference throughout this specification to "an embodiment" or "the embodiment" means that a particular feature, structure, or characteristic described in connection with that embodiment is included in at least one embodiment. Thus, the quoted phrases, or variations thereof, as recited throughout this specification are not necessarily all referring to the same embodiment.

Similarly, it should be appreciated that in the above description of embodiments, various features are sometimes grouped together in a single embodiment, figure, or description thereof for the purpose of streamlining the disclosure. This method of disclosure, however, is not to be interpreted as reflecting an intention that any claim require more features than those expressly recited in that claim. Rather, as the following claims reflect, inventive aspects lie in a combination of fewer than all features of any single foregoing disclosed embodiment.

Without further elaboration, it is believed that one skilled in the art can use the preceding description to utilize the invention to its fullest extent. The claims and embodiments disclosed herein are to be construed as merely illustrative and exemplary, and not a limitation of the scope of the present disclosure in any way. The scope of the invention is therefore defined by the following claims.

## Claims

1. A system (100) for performing dilation, comprising:
a catheter body (110) comprising:
an inflation lumen (111) extending along a portion of the catheter body (110);
a fluid connector (137) coupled to the catheter body (110) adjacent a proximal end and in fluid communication with the inflation lumen (111); and
a balloon (120) comprising a distal end and a proximal end, wherein the proximal end is coupled to a distal end (114) of the catheter body (110),
**characterized in that** the balloon (120) is configured to be expanded to four or more predefined discrete diameters correlated to four or more predefined discrete pressures; and
a label displaying the four or more predefined discrete diameters and the correlated four or more predefined discrete pressures.

2. The system (100) of claim 1, wherein the balloon (120) is expandable to five predefined discrete diameters.

3. The system (100) of claim 1, wherein the balloon (120) is expandable to more than four predefined discrete diameters.

4. The system (100) of any one of claims 1 to 3, further comprising a support wire (158), wherein the support wire is (158) disposed within the inflation lumen (111) and extends distally from the distal end (114) of the catheter body.

5. The system (100) of claim 4, wherein the support wire (158) is configured to restrict longitudinal compression of the balloon (120).

6. The system (100) of claim 4 or 5, wherein the support wire (158) comprises radiopaque markers disposed between the proximal end and the distal end of the balloon (120).

7. The system (100) of any one of claims 1 to 6, wherein the balloon (120) is comprised of one of nylon, polyether block amide, and polyurethane.

8. The system (100) of any one of claims 1 to 7, wherein the balloon (120) is expandable to diameters ranging from 1.0 mm to 25.0 mm.

9. The system (100) of any one of claims 1 to 8, wherein the balloon (120) is expandable at pressures ranging from one atmosphere to twelve atmospheres.

10. The system (100) of any one of claims 1 to 9, wherein the label is coupled to the system (100) or included with packaging materials.

11. The system (100) of any one of claims 1 to 10, wherein the catheter body (110) is configured to pass through a working lumen (152) of an elongated member (150).

12. The system (100) of claim 11, wherein the elongated member (150) comprises an endoscope.

13. The system (100) of any one of claims 1 to 12, further comprising a balloon inflation device (130) configured to be coupled to the fluid connector (137) and comprising a pressure gauge (135).

14. The system (100) of any one of claims 1 to 13, wherein the inflation lumen (111) comprises an opening at a distal end.

## Patentansprüche

1. System (100) zum Ausführen von Dilatation, umfassend:
einen Katheterkörper (110), umfassend:
ein Aufblaslumen (111), das sich entlang eines Abschnitts des Katheterkörpers (110) erstreckt;
einen Fluidverbinder (137), der einem proximalen Ende benachbart an den Katheterkörper (110) gekoppelt ist und mit dem Aufblaslumen (111) in Fluidverbindung steht; und
einen Ballon (120), der ein distales Ende und ein proximales Ende umfasst, wobei das proximale Ende an ein distales Ende (114) des Katheterkörpers (110) gekoppelt ist,
**dadurch gekennzeichnet, dass** der Ballon (120) dazu konfiguriert ist, auf vier oder mehr vordefinierte diskrete Durchmesser ausgedehnt zu werden, die mit vier oder mehr vordefinierten Drücken korrelieren; und
ein Etikett, das die vier oder mehr vordefinierten diskreten Durchmesser und die korrelierten vier oder mehr vordefinierten diskreten Drücke angibt.

2. System (100) nach Anspruch 1, wobei der Ballon (120) auf fünf vordefinierte diskrete Durchmesser ausdehnbar ist.

3. System (100) nach Anspruch 1, wobei der Ballon (120) auf mehr als vier vordefinierte diskrete Durchmesser ausdehnbar ist.

4. System (100) nach einem der Ansprüche 1 bis 3, ferner umfassend einen Stützdraht (158), wobei der Stützdraht (158) in dem Aufblaslumen (111) angeordnet ist und sich von dem distalen Ende (114) des Katheterkörpers nach distal erstreckt.

5. System (100) nach Anspruch 4, wobei der Stützdraht (158) dazu konfiguriert ist, die Längsstauchung des Ballons (120) einzuschränken.

6. System (100) nach Anspruch 4 oder 5, wobei der Stützdraht (158) röntgendichte Markierungen umfasst, die zwischen dem proximalen Ende und dem distalen Ende des Ballons (120) angeordnet sind.

7. System (100) nach einem der Ansprüche 1 bis 6, wobei der Ballon (120) aus einem von Nylon, Polyetherblockamid und Polyurethan besteht.

8. System (100) nach einem der Ansprüche 1 bis 7, wobei der Ballon (120) auf Durchmesser im Bereich von 1,0 mm bis 25,0 mm ausdehnbar ist.

9. System (100) nach einem der Ansprüche 1 bis 8, wobei der Ballon (120) bei Drücken im Bereich von einer Atmosphäre bis zwölf Atmosphären ausdehnbar ist.

10. System (100) nach einem der Ansprüche 1 bis 9, wobei das Etikett an das System (100) gekoppelt ist oder mit Verpackungsmaterialien enthalten ist.

11. System (100) nach einem der Ansprüche 1 bis 10, wobei der Katheterkörper (110) dazu konfiguriert ist, durch ein Arbeitslumen (152) eines langgestreckten Elements (150) zu gelangen.

12. System (100) nach Anspruch 11, wobei das langgestreckte Element (150) ein Endoskop umfasst.

13. System (100) nach einem der Ansprüche 1 bis 12, ferner umfassend eine Ballonaufblasvorrichtung (130), die dazu konfiguriert ist, an den Fluidverbinder (137) gekoppelt zu werden, und ein Druckmessgerät (135) umfasst.

14. System (100) nach einem der Ansprüche 1 bis 13, wobei das Aufblaslumen (111) eine Öffnung an einem distalen Ende umfasst.

## Revendications

1. Système (100) servant à des fins de dilatation, comportant :
un corps de cathéter (110) comportant :
une lumière de gonflage (111) s'étendant le long d'une partie du corps de cathéter (110) ;
un raccord de fluide (137) accouplé au corps de cathéter (110) de manière adjacente par rapport à une extrémité proximale et en communication fluidique avec la lumière de gonflage (111) ; et
un ballonnet (120) comportant une extrémité distale et une extrémité proximale, dans lequel l'extrémité proximale est accouplée à une extrémité distale (114) du corps de cathéter (110),
**caractérisé en ce que** le ballonnet (120) est configuré pour s'étendre jusqu'à quatre diamètres discrets prédéfinis ou plus corrélés à quatre pressions discrètes prédéfinies ou plus ; et
une étiquette affichant les quatre diamètres discrets prédéfinis ou plus et les quatre pressions discrètes prédéfinies ou plus corrélées.

2. Système (100) selon la revendication 1, dans lequel le ballonnet (120) est en mesure de s'étendre jusqu'à cinq diamètres discrets prédéfinis.

3. Système (100) selon la revendication 1, dans lequel le ballonnet (120) est en mesure de s'étendre jusqu'à plus de quatre diamètres discrets prédéfinis.

4. Système (100) selon l'une quelconque des revendications 1 à 3, comportant par ailleurs un fil de support (158), dans lequel le fil de support (158) est disposé à l'intérieur de la lumière de gonflage (111) et s'étend de manière distale depuis l'extrémité distale (114) du corps de cathéter.

5. Système (100) selon la revendication 4, dans lequel le fil de support (158) est configuré pour restreindre toute compression longitudinale du ballonnet (120).

6. Système (100) selon la revendication 4 ou la revendication 5, dans lequel le fil de support (158) comporte des marqueurs radio-opaques disposés entre l'extrémité proximale et l'extrémité distale du ballonnet (120).

7. Système (100) selon l'une quelconque des revendications 1 à 6, dans lequel le ballonnet (120) est constitué de l'un parmi du nylon, du polyéther bloc amide, et du polyuréthane.

8. Système (100) selon l'une quelconque des revendications 1 à 7, dans lequel le ballonnet (120) est en mesure de s'étendre jusqu'à des diamètres allant de 1,0 mm à 25,0 mm.

9. Système (100) selon l'une quelconque des revendications 1 à 8, dans lequel le ballonnet (120) est en mesure de s'étendre jusqu'à des pressions allant d'une atmosphère à douze atmosphères.

10. Système (100) selon l'une quelconque des revendications 1 à 9, dans lequel l'étiquette est accouplée au système (100) ou est comprise avec les matériaux de conditionnement.

11. Système (100) selon l'une quelconque des revendications 1 à 10, dans lequel le corps de cathéter (110) est configuré pour passer au travers d'une lumière de travail (152) d'un élément allongé (150).

12. Système (100) selon la revendication 11, dans lequel l'élément allongé (150) comporte un endoscope.

13. Système (100) selon l'une quelconque des revendications 1 à 12, comportant par ailleurs un dispositif de gonflage de ballonnet (130) configuré pour être accouplé au raccord de fluide (137) et comportant un manomètre (135).

14. Système (100) selon l'une quelconque des revendications 1 à 13, dans lequel la lumière de gonflage (111) comporte une ouverture au niveau d'une extrémité distale.
